(19) Europäisches Patentamt
European Patent Office
Office européen des brevets

(11) Veröffentlichungsnummer : **0 037 975**
**B1**

(12) # EUROPÄISCHE PATENTSCHRIFT

(45) Veröffentlichungstag der Patentschrift : **04.07.84**

(51) Int. Cl.[3] : **C 09 B 49/10**, C 09 B 53/02, C 07 D209/88, D 06 P 1/30

(21) Anmeldenummer : **81102495.9**

(22) Anmeldetag : **02.04.81**

(54) **Neue Schwefelfarbstoffe der Carbazolreihe, ihre Herstellung und Verwendung sowie neue Zwischenprodukte.**

(30) Priorität : **11.04.80 DE 3013994**

(43) Veröffentlichungstag der Anmeldung : **21.10.81 Patentblatt 81/42**

(45) Bekanntmachung des Hinweises auf die Patenterteilung : **04.07.84 Patentblatt 84/27**

(84) Benannte Vertragsstaaten : **CH DE FR GB IT LI NL**

(56) Entgegenhaltungen :
**DE-C- 235 836**
**DE-C- 400 565**
**DE-C- 432 177**
**FR-A- 427 900**
**FR-A- 457 535**
**GB-A- 15 949**

(73) Patentinhaber : **CASSELLA Aktiengesellschaft**
**Hanauer Landstrasse 526**
**D-6000 Frankfurt am Main 61 (DE)**

(72) Erfinder : **Piesch, Steffen, Dr.**
**An der Heide 32**
**D-6370 Oberursel/Taunus (DE)**
Erfinder : **Weidemüller, Wolf, Dr.**
**Nordring 101**
**D-6000 Frankfurt am Main 60 (DE)**

(74) Vertreter : **Urbach, Hans-Georg, Dr. et al**
**Hanauer Landstrasse 526**
**D-6000 Frankfurt am Main 61 (DE)**

**EP 0 037 975 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Schwefelfarbstoffe, die hergestellt werden können durch Schwefeln neuer Leucoindophenole bzw. -indamine der Formel I

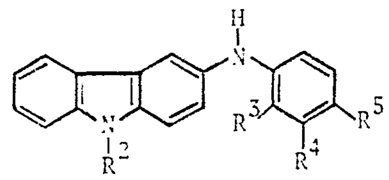

(I)

worin die Symbole $R^2$ bis $R^5$ die in Anspruch 1 angegebenen Bedeutungen haben, nach an sich bekannten Verfahren, ihre Verwendung zum Färben von Zellulose und Polyamid sowie die zur Herstellung der neuen Farbstoffe benötigten neuen Leucoindophenole und Leucoindamine der Formel I.

Es ist bekannt, durch Umsetzung geeigneter organischer Verbindungen mit Schwefel und gegebenenfalls Alkalisulfiden oder Polysulfiden bei erhöhter Temperatur Schwefelfarbstoffe herzustellen. Eine knappe Übersicht über die wichtigsten Gruppen bisher bekannter, geeigneter Ausgangsmaterialien findet sich beispielsweise in Kirk-Othmer, « Encyclopedia of Chemical Technologie », 2. Auflage, Bd. 19, Seiten 425 bis 435. Gemäß der großen technischen Bedeutung der Schwefelfarbstoffe existiert eine große Anzahl von Veröffentlichungen, die sowohl die allgemeinen Grundzüge der verschiedenen Schwefelungsverfahren, wie « Backschmelze » und « Kochschmelze » betreffen, als auch einzelnen Gruppen geeigneter Ausgangsmateriallien und Schwefelfarbstoffen gewidmet sind.

Im Zusammenhang mit vorliegender Erfindung erscheinen insbesondere die Monographien: O. Lange « Die Schwefelfarbstoffe, ihre Herstellung und Verwendung », 2. Ausgabe, Spamer, Leipzig (1925) sowie K. Venkataraman « The Chemistry of Synthetic Dyes and Pigments », Bd. 2, Academic Press, Inc., New York (1952), Kapitel 35, von Interesse.

Aus einer ganzen Reihe von Patenten, z. B. der deutschen Patentschrift 218 371, dem britischen Patent 2918/09, der US-Patentschrift 919 572 sowie den betreffenden Abdrucken in « Friedländer » Band 10, Seiten 256, 258, 71 bis 74 und 75 bis 81, ist es bekannt, Leuco-Carbazolindophenole der Formel

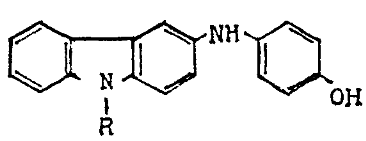

worin R Wasserstoff oder Ethyl ist, zu den blauen Schwefelfarbstoffen C. I. Sulfur Blue 42 und 43 zu schwefeln.

Weitere Derivate dieses Ausgangsmaterials sind bislang nicht zu Schwefelfarbstoffen verarbeitet worden, obwohl die oben genannten C. I. Sulfur Blue 42 und 43 ausgezeichnete Eigenschaften haben und bereits seit 70 Jahren bekannt sind. Zicht man in Betracht, daß Farbstoffe dieses Typs auch in anderen als blauen Nuancen von größtem Interesse gewesen wären, so erkennt man, daß alle Anstrengungen, das Gebiet der Carbazolindophenol-Schwefelfarbstoffe auszubauen, bisher gescheitert sind.

Ein wesentliches Moment für diese Mißerfolge dürfte darin zu erblicken sein, daß bislang keine ausreichend variablen und technisch einfachen und somit preisgünstigen Verfahren zur Herstellung der Leuco-Carbazolindophenole bekannt waren. Diese Verbindungen wurden durch Reduktion der entsprechenden Carbazolindophenole (Carbazol-3-iminochinone bzw. Benzochinon-(1,4)-mono-carbazolyl-(3)-imide) hergestellt, für deren Herstellung bis jetzt im wesentlichen zwei Verfahren bekannt geworden sind:

1. Durch Kondensation von Carbazol bzw. von N-substituierten Carbazolen mit p-Nitrosophenol in kalter, hochprozentiger Schwefelsäure unter Kühlung (FR-PS 457 535, deutsche Reichspatentschriften 218 371, 224 951 und 230 119).

Nach diesem Verfahren werden auch die aus der Deutschen Patentschrift Nr. 235836 bekannten chlorsubstituierten Carbazolindophenole und deren Leuco-Verbindungen hergestellt, die beim Schwefeln nach den üblichen Verfahren blaue Schwefelfarbstoffe liefern. Hierbei ergibt sich prinzipiell der erhebliche Nachteil, daß nicht nur die Kondensation des Nitrosophenols mit dem Aminocarbazolderivat in Schwefelsäure als Lösungsmittel durchgeführt wird, sondern auch die erforderlichen Nitrosophenolderivate durch Nitrosierung der entsprechenden Phenole in einem sehr großen Überschuß von Schwefelsäure als Lösungsmittel hergestellt werden.

Insbesondere die bei der Nitrosierung anfallende Säuremenge ist zunächst zu reinigen und dann auf ökologisch einwandfreie Weise zu beseitigen, was dieses Verfahren erheblich verteuert. Die zusätzliche Kostenbelastung durch den Einsatz von halogensubstituierten Carbazolen bzw. Phenolen verteuert die halogensubstituierten Carbazolindophenole und ihre Leukoverbindungen so erheblich, daß die daraus

herstellbaren Schwefelfarbstoffe bislang nicht in technischen Ausmaß hergestellt worden sind.

2. Durch Umsetzung von Carbazol und seiner Derivate mit p-Aminophenol in konzentrierter Schwefelsäure unter Kühlung in Gegenwart eines Oxidationsmittels, wie Mangandioxid (FR-PS 457 535).

Die anschließende Reduktion der Carbazolindophenole zu den Leucoverbindungen erfolgt im allgemeinen in verdünnter Säure mit Eisen.

3. Zur Herstellung bestimmter Derivate der Verbindungen der allgemeinen Formel I ist es auch bekannt (FR-PS 390 715), Derivate des 2,2'-Dinitrophenyls zu reduzieren und durch Behandlung mit verdünnten, kochenden Mineralsäuren einen Ringschluß zum Carbazolderivat herbeizuführen.

4. In der Britischen Patentschrift Nr. 15 949 aus dem Jahre 1914 wird dargelegt, daß es möglich sein soll, Leuco-indamine der Formel

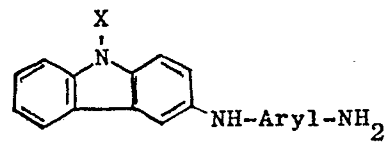

herzustellen indem man Carbazol oder N-substituiertes Carbazol in Gegenwart konzentrierter Schwefelsäure mit Arylsulfimidoarylenchlorimiden der Formel

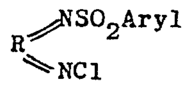

worin R ein Arylenrest der Benzol- oder Naphthalinreihe ist, kondensiert. Nach den Angaben dieser Druckschrift läßt sich das so erhaltene Leucoindamin in üblicher Weise schwefeln unter Bildung eines Schwefelfarbstoffs der sich zum Teil in Alkalisulfid-Lösung, zum Teil in Alkalihyposulfit-Lösung auflösen läßt und aus diesen Lösungen Baumwolle intensiv blau färbt. Der so erhaltene Schwefelfarbstoff ist offensichtlich sehr uneinheitlich (darauf deuten die unterschiedlichen Löslichkeiten der Farbstoffanteile) und die Farbnuance blau deutet auch darauf hin, daß im Verlauf der Umsetzungen des Arylsulfimidoarylenchlorimids mit dem Carbazol eine Hydrolyse eintritt und in Wirklichkeit nicht das Leucoindamin der oben angegobenen Formel, sondern ein mehr oder weniger werunreinigtes Leuco-indophenol der Formel

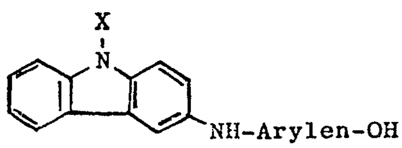

geschwefelt wurde so daß auch bei diesem Prozeß im wesentlichen die blauen Schwefelfarbstoffe vom Typ des C. I. Sulfur Blue 42 erhalten wurden.

Der Ersatz der OH-Gruppe des Indophenols durch —NH sollte nämlich zu einer merklichen Farbverschiebung nach grün führen.

In der Tat zeigt die Herstellung des Indamins nach dem erfindungsgemäßen Verfahren und anschließende Schwefelung, daß ein Schwefelfarbstoff erhalten wird, der Baumwolle blaustichig grün färbt, und somit die erwartete Farbverschiebung tatsächlich eintritt.

In O. Lange « Die Zwischenprodukte der Teerfarbenfabrikation », Leipzig 1920, wird auf Seite 315 auf die deutsche Patentanmeldung A 25 495, Klasse 12 P, vom 24.2.1914 hingewiesen, in der Herstellung der Indophenole und ihrer Leuco-Verbindungen aus 3-Aminocarbazol oder seinen N-substituiertenn Derivaten durch Kondensation mit Hydrochinon in Gegenwart wasserentziehender Mittel unter Vermeidung der Oxidation beschrieben sein soll. Die Anmeldung wurde nicht veröffentlicht, so daß Details dieses Verfahrens nicht bekannt geworden sind. Bei der Durchführung der Reaktion in Gegenwart der damals bekannten wasserentziehenden Mittel, nämlich konzentrierter Schwefelsäure oder Zinkchlorid, ist die Aufarbeitung durch Wasser zwingend notwendig, und man erhält das Leuco-indophenol nur in geringer Ausbeute neben einer Reihe von unerwünschten Nebenprodukten. Die Reinigung der so erhaltenen Leuco-indophenole gestaltet sich schwierig und schließt die Anwendung dieses Verfahrens für die technische Herstellung der gewünschten Produkte aus. Auch die oben unter 2. und 3. genannten bekannten Herstellungsverfahren liefern die gewünschten Indophenole bzw. Leuco-indophenole nicht in der gewünschten Ausbeute und Reinheit und konnten im Laufe der langen Zeit, seit der sie bekannt wurden, nicht soweit verbessert werden, daß sie technisch durchgeführt werden. Als optimales bekanntes Verfahren hat sich bisher in der Technik das oben unter 1. genannte Kondensationsverfahren behauptet, bei dem zunächst p-Nitrosophenol mit dem Carbazolderivat kondensiert wird und das erhaltene chinoide Zwischenprodukt des Indophenols anschließend in der Regel reduziert werden muß. da das chinoide Produkt besonders instabil ist.

Dieses technische Verfahren ist auf die Herstellung von 3-(4-Hydroxyphenylamino)-carbazol und seinem 9-Ethylderivat beschränkt; es ist nicht gelungen, es auf die Herstellung von anderen Derivaten dieser Verbindung auszudehnen.

Nach einem älteren Vorschlag (deutsche Patentanmeldung P 29 00 441.9) können diese Verbindungen jedoch auch dadurch erhalten werden, daß ein 3-Aminocarbazol der allgemeinen Formel

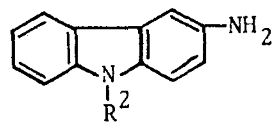

mit p-Hydrochinon oder p-Aminophenol oder mit einem Gemisch aus p-Hydrochinon und p-Aminophenol in mindestens stöchiometrischen Mengen unter Ausschluß von Sauerstoff auf Temperaturen von 150 bis 300 °C erhitzt wird.

Es wurde nun gefunden, daß es gelingt, die Palette der Schwefelfarbstoffe um weitere sehr ansprechende und gesuchte grüne, olive und blaue Nuancen zu bereichern und Schwefelfarbstoffe mit für diese Klasse ausgezeichneten Echtheiten und sehr vorteilhaften Applikations-möglichkeiten herzustellen, wenn man neue Indophenole bzw. Indamine der Formel I

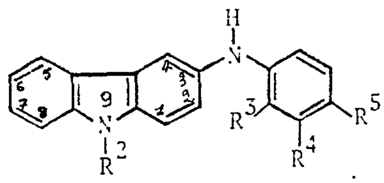

(I)

worin $R^2$ Wasserstoff oder Alkyl mit 1 bis 8 C-Atomen, $R^3$ und $R^4$ unabhängig voneinander Wasserstoff oder Alkyl mit 1 bis 4 C-Atomen bedeuten, oder gemeinsam ein Strukturelement der Formel Ia bis d

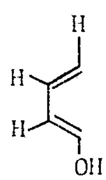 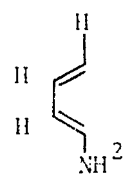 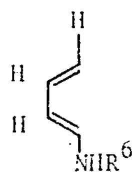 oder 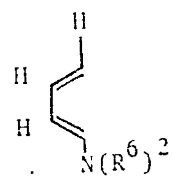

(Ia) (Ib) (Ic) (Id)

bilden, $R^5$ Alkoxy mit 1 bis 4 C-Atomen, Amino, $-NHR^6$, $-N(R^6)_2$,

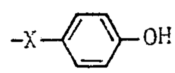

und, sofern $R^3$ und $R^4$ gemeinsam ein Strukturelement der Formel Ia bis d bilden, auch Wasserstoff und, sofern $R^3$ und/oder $R^4$ ungleich Wasserstoff sind, auch Hydroxy, $R^6$ Alkyl mit 1 bis 4 C-Atomen und X $-S-$, $-SO_2-$, $-NH-$ oder eine direkte Bindung bedeuten, nach an sich bekannten Verfahren, vorzugsweise nach dem Verfahren der « Kochschmelze », schwefelt.

Alkylreste, für die $R^2$, $R^3$, $R^4$ oder $R^6$ steht, können linear oder verzweigt und gegebenenfalls durch ein Halogenatom, insbesondere ein Chloratom, substituiert sein. Bevorzugt sind lineare, unsubstituierte Alkylreste.

Besonders vorteilhafte Farbstoffe werden erhalten, wenn Leucoindophenole bzw. -indamine der Formel I geschwefelt werden, in denen $R^2$ Wasserstoff oder Alkyl mit 1 bis 4 C-Atomen; eines der Symbole $R^3$ oder $R^4$ Wasserstoff, das andere Wasserstoff, oder Alkyl mit 1 bis 4 C-Atomen bedeuten; oder $R^3$ und $R^4$ gemeinsam ein Strukturelement der Formel Ia oder Ib bilden, und $R^5$ Wasserstoff ist, oder $R^5$ Alkoxy mit 1 bis 4 C-Atomen, Amino, $-NHR^6$, $-N(R^6)_2$ oder

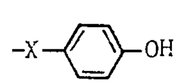

bedeuten.

Die Verbindunggen der Formel I können auch dann zu erfindungsgemäßen Schwefelfarbstoffen geschwefelt werden, wenn sie die bei der Herstellung anfallenden Isomeren, bei denen die Phenylaminogruppe in der 1-Stellung des Carbazolsystems gebunden ist, in den technisch üblichen Anteilen von bis zu ca. 25 % enthalten.

Das Schwefeln der Verbindungen I in der Kochschmelze erfolgt in der an sich bekannten Weise durch Reaktion mit Schwefel und/oder Alkalipolysulfid in wäßrigem Medium oder einem organischen Lösungsmittel bei erhöhter Temperatur, wie es z. B. bei O. Lange « Die Schwefelfarbstoffe, ihre Herstellung und Verwendung » (1912), Seiten 208 bis 220 sowie 223 bis 225, oder in Venkataraman, « The Chemistry of Synthetic Dyes », Bd. 2 (1952), Seite 1062 ff. und Seite 1103 ff., sowie Bd. 7 (1974), Seite 24 ff., Academic Press, New York, San Francisco, London, beschrieben ist.

Besonders geeignet sind Kochschmelzen, die unter Verwendung von Alkalipolysulfid arbeiten, wobei pro Mol des zu schwefelnden Produkts 0,1 bis 10 Mole, vorzugsweise 0,5 bis 4 Mole Natriumsulfid und 1 bis 30 Mole, vorzugsweise 2 bis 20 Mole Schwefel eingesetzt werden. Sie werden in den für Schwefelschmelzen üblichen Lösungsmitteln durchgeführt. Als Lösungsmittel, in denen zweckmäßigerweise gearbeitet werden kann, sind Alkanole oder Cycloalkanole mit 1 bis 7 C-Atomen, wie z. B. Methanol, Ethanol, Propanol, Isopropanol, n-Butanol, Isobutanol, Amylalkohol, Cyclohexanol, Methylcyclohexanol und Monoalkyläther, insbesondere Monoalkyläther von Ethylenglykol und Diethylenglykol, wie z. B. Ethylenglykolmonomethyläther, -mono-ethyläther, -mono-propylether, Diethylenglykol-mono-methyläther, -mono-ethyläther und -mono-propyläther geeignet.

Die Schwefelung der Verbindungen der Formel I kann unter Ausschluß von Wasser oder in rein wäßrigem Medium erfolgen, wird aber normalerweise in wasserhaltigen Lösungsmitteln mit einem Wassergehalt von 5 bis 70 % durchgeführt. Im Falle der mit Wasser mischbaren Lösungsmittel arbeitet man vorzugsweise unter Verwendung der üblichen hydrotropen Verbindungen, z. B. des Natriumsalzes der Xylolsulfonsäure.

Die Kochschmelze erfolgt bei Temperaturen von 80 bis 300 °C, vorzugsweise 110 bis 240 °C, insbesondere im Bereich von 120 bis 170 °C, und zweckmäßigerweise bei einer Reaktionsdauer von 1 bis 100, vorzugsweise von 20 bis 80 Stunden.

Unter Inkaufnahme der bekannten Nachteile der sogenannten « Backschmelzen » oder « Schwefelschmelzen » (vgl. O. Lange « Die Schwefelfarbstoffe » (1912) Seiten 221 und 222) können die Verbindungen der Formel I auch nach diesem Verfahren zu Schwefelfarbstoffen geschwefelt werden.

Bei der Herstellung der erfindungsgemäßen Schwefelfarbstoffe durch Backschmelze werden diese mit der 7- bis 30-fachen Molmenge Schwefel und der 2- bis 6-fachen Molmenge Natriumsulfid 1 bis 20 Stunden auf 200 bis 300 °C erhitzt. Auch hierbei kann zunächst im Lösungsmittel, vorzugsweise in Wasser, gearbeitet werden, um eine gute Homogenität des Reaktionsansatzes zu gewährleisten. Das Lösungsmittel wird dann abgegampft und die lösungsmittelfreie Schmelze zweckmäßigerweise in Schutzgasatmosphäre auf die gewünschte Temperatur erwärmt.

Die nach dem erfindungsgemäßen Verfahren hergestellten Schwefelfarbstoffe können auf übliche Weise isoliert und danach in die verschiedenen Handelsformen gebracht werden.

Wurde die Schwefelung in einem mit Wasser mischbaren Lösungsmittel in Gegenwart von hydrotropen Verbindungen ausgeführt, so kann die Schmelze auch ohne Isolierung des Farbstoffs direkt zu flüssigen gebrauchsfertigen Farbstoffen weiterverarbeitet werden.

Die erfindungsgemäß herstellbaren Schwefelfarbstoffe lassen sich durch Reduktionsmittel, wie sie zur Reduktion von Schwefelfarbstoff üblich sind, sehr gut reduzieren und ergeben so klare wäßrige Lösungen. Sie können zum Färben von vorzugsweise pflanzlichen Fasern nach den üblichen, für das Färben mit Schwefel- oder Schwefelküpenfarbstoffen bekannten Verfahren eingesetzt werden.

Hierzu werden sie mit Reduktionsmitteln, vorzugsweise Natriumdithionit, Natriumsulfid oder Natriumhydrogensulfid, möglicherweise aber auch mit Natriumformaldehyd-sulfoxylat, Glukose oder organischen Mercaptoverbindungen, wie z. B. Thioglycerin oder Thioglycolsäure, in die lösliche Leucoform überführt, die auf die Fasern aufzieht. Die in flüssiger gebrauchsfertiger Form vorliegenden Farbstoffen enthalten die lösliche Leucoform und Reduktionsmittel und können auch ohne weiteren Zusatz von Reduktionsmitteln zum Färben der genannten Fasern verwendet werden.

Nach dem Aufziehen der Farbstoffe auf die Fasern wird die Leucoform der erfindungsgemäßen Schwefelfarbstoffe in üblicher Weise, beispielsweise durch « Verhängen » der Färbungen an der Luft oder Oxidation mit Oxidationsmitteln, wie beispielsweise Wasserstoffperoxid, Alkalibichromat, Alkalichlorit oder Alkalijodat wieder in die unlösliche Form des Schwefelfarbstoffs überführt. Die erfindungsgemäß herstellbaren Schwefelfarbstoffe färben die Zellulose in vollen grünen, blaugrünen, oliven und blauen Tönen und stellen damit eine überaus wertvolle Ergänzung der in den coloristischen Eigenschaften vergleichbaren Carbazolschwefelfarbstoffen C. I. Sulfur Blue 42 und 43 dar.

Auf Zellulosefasern appliziert, haben die erfindungsgemäßen Farbstoffe gegenüber den im Farbton nächstliegenden Handelsprodukten Sulphur Green 2, C. I. Nr. 53571, und Sulphur Green 3, C. I. Nr. 53570, insbesondere folgende Vorteile : sehr gute Lichtechtheit, sehr gute Naßechtheiten, sehr gute Peroxidwaschechtheit nach DIN 54 015 und gute Mercerisierechtheit.

Ein weiterer Vorteil der erfindungsgemäßen Farbstoffe ist ihre Stabilität gegen Überreduktion bei der Einwirkung von Alkalidithionit oder Alkali-Formaldehyd-sulfoxylat sowie ihre einfache technische Herstellbarkeit.

Auch Polyamid läßt sich mit den erfindungsgemäßen Schwefelfarbstoffen färben. Man erhält je nach Reduktionsmittel und eingesetzter Farbstoffmenge grüne bis schwarze Farbtöne.

Die zur Herstellung der erfindungsgemäßen neuen Schwefelfarbstoffe einzusetzenden Indophenole und Indamine der allgemeinen Formel I sind ebenfalls neu.

Sie werden erhalten, indem ein 3-Aminocarbazol der allgemeinen Formel II

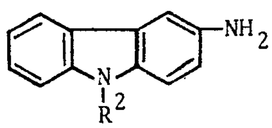

(II)

worin $R^2$ die oben angegebenen Bedeutungen hat, mit einem Phenol bzw. Naphthol oder Aminobenzol bzw. Aminonaphthalin der Formel III

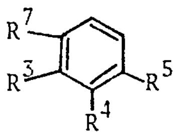

(III)

worin $R^7$ eine der Gruppen —OH oder —$NH_2$ ist und $R^3$, $R^4$ und $R^5$ die oben genannten Bedeutungen haben, in mindestens stöchiometrischen Mengen unter Ausschluß von Sauerstoff auf Temperaturen von 150 bis 300 °C, zweckmäßigerweise auf 180 bis 280 °C, erhitzt wird.

Unter den technischen Isomerengemischen der 3-Aminocarbazole der Formel II sind solche Produkte zu verstehen, die alle bei der Herstellung der 3-Aminocarbazole im technischen Maßstab entstehenden isomeren Aminocarbazole enthalten. Derartige rohe, technische Produkte enthalten neben dem Hauptprodukt, den 3-Aminocarbazolen, vorwiegend die entsprechenden 1-Aminocarbazole.

Demgemäß enthalten die erfindungsgemäßen Leucoindophenole bzw. -indamine der Formel I, die, ausgehend von technischen, rohen 3-Aminocarbazolen erhalten werden, in der Regel auch Ansteile der isomeren Verbindungen, die die substituierte Anilinogruppe in der 1-Stellung des Carbazolgerüstes enthalten.

Aus Arbeiten von R. Gnehm, veröffentlicht im Journal f. prakt. Chemie, Bd. 69, Seiten 161 bis 162, ist es bekannt, p'-Dimethylamino-p-hydroxydiphenylamin durch Umsetzung von N,N-Dimethyl-p-phenylendiamin mit Hydrochinon in Gegenwart von Zinkchlorid herzustellen. Die Methode ist jedoch für technische Belange völlig unzulänglich, denn die Ausbeute kann selbst bei intensiven Optimierungsbemühungen nicht über ca. 35 % d. Th. gesteigert werden. Nebenher fallen große Mengen von Abfallprodukten an, deren Beseitigung zusätzliche erhebliche Belastungen mit sich bringt. Die gleichen prohibitiven Nachteile zeigt die vom gleichen Autor (J. f. prakt. Chemie, Bd. 69, Seite 223) untersuchte Herstellung von p-Dimethylamino-p'-amino-diphenylamin. Auch hierbei wird die Kondensation in Gegenwart von Zinkchlorid vorgenommen, weil ohne dieses Kondensationsmittel kein einheitlicher Reaktionsverlauf zu erzielen war.

Es war daher sehr überraschend, daß die erfindungsgemäße Durchführung der Kondensation der Verbindungen der Formeln II und III auf so einfache Weise in ausgezeichneten Ausbeuten zu den Verbindungen der Formel I führt.

Bei der Durchführung des erfindungsgemäßen Verfahrens werden im einfachsten Fall die Reaktionsteilnehmer zusammengeschmolzen, gegebenenfalls mit den Verbindungen der Formel III im Überschuß und so lange auf der oben genannten Kondensationstemperatur, vorzugsweise bei 220 bis 260 °C, gehalten, bis kein Reaktionswasser oder Ammoniak aus dem Ansatz mehr abdestilliert. Je nach der gewählten Reaktionstemperatur ist die Reaktion in der Regel nach 2 bis 10 Stunden praktisch abgeschlossen. Die Aufarbeitung des Reaktionsgemisches erfolgt im einfachsten Fall durch Abdestillieren eines eventuellen Überschusses der Verbindungen der Formel III unter vermindertem Druck und anschließendes Ablassen der Schmelze aus dem Reaktionsgefäß und nachfolgendes Zerkleinern in einer geeigneten Mühle. Eine andere bequeme Möglichkeit, überschüssige Verbindungen der Formel III, die eine —OH—Gruppe enthalten, aus dem Reaktionsprodukt zu entfernen, ist die Behandlung des Produkts mit wäßrigen Alkalihydroxidlösungen.

In vielen Fällen ist es möglich, zum Zwecke der Reinigung die entstandene Verbindung der Formel I zu destillieren, was direkt aus dem Reaktionsgefäß heraus geschehen kann. Diese Destillation wird zweckmäßigerweise unter vermindertem Druck vorgenommen. Es war außerdeordentlich überraschend, daß Verbindungen der Formel I, die bisher als außerordentlich empfindlich und leicht zersetzlich angesehen wurden, sich durch eine derartige Herstellung und Destillation auf einfachstem Wege reinigen lassen. Selbst bei Sumpftemperaturen von 300 °C und mehr findet praktisch keine Zersetzung statt.

Die Kondensationsreaktion zwischen den Aminocarbazolen der Formel II und der Verbindung der Formel III kann durch einen Zusatz einer katalytischen Menge von Jod deutlich beschleunigt werden und führt zusätzlich zu reineren Produkten. Als katalytische Mengen kommen normalerweise Zusätze von 0,1

bis 4, vorzugsweise 0,1 bis 1 Gew.%, bezogen auf das eingesetzte Carbazolderivat der allgemeinen Formel II, in Betracht. Da der Jodzusatz weder bei der Aufarbeitung der erfindungsgemäß hergestellten Leucoindophenole, noch bei deren Weiterverarbeitung stört, ist die Durchführung des erfindungsgemäßen Verfahrens unter Zusatz von katalytischen Mengen Jod bevorzugt.

Die erfindungsgemäße Kondensation der Verbindungen der Formeln II und III kann auch in Gegenwart eines inerten organischen Lösungsmittels durchgeführt werden. Zweckmäßigerweise werden solche Lösungsmittel verwendet, deren Siedepunkt oberhalb der Kondensationstemperatur, d. h. insbesondere oberhalb von 150 bis 260 °C liegt. Sofern aufgrund besonderer Überlegungen Lösungsmittel eingesetzt werden sollen, deren Siedepunkt tiefer als 150 °C liegt, ist zweckmäßigerweise in einem druckfest verschlossenen Gefäß (Autoklav) zu arbeiten. Organische Lösungsmittel, die für die Durchführung des erfindungsgemäßen Verfahrens geeignet sind, sind z. B. Erdölfraktionen mit einem Siedepunkt über 150 °C, cyclische Kohlenwasserstoffe, wie beispielsweise Decalin, oder aromatische Lösungsmittel, insbesondere das technische Gemisch aus Monochlor- und Dichlorbenzol. Insbesondere wenn die hergestellten Leucoindophenole anschließend in einer sogenannten Kochschmelze, d. h. einer Lösung von Natriumpolysulfiden, in Schwefelfarbstoffe überführt werden, kann die erfindungsgemäße Kondensation der Verbindung der Formel III mit einem Carbazolderivat der allgemeinen Formel II auch in Gegenwart eines Lösungsmittels der allgemeinen Formel IV oder V

$$R^8O—(CH_2CH_2O)_n—R^8 \quad (IV) \quad R^8O—(CH_2CH_2O)_n—H \quad (V)$$

worin $R^8 = —CH_3$, $—C_2H_5$, $—C_3H_7$ und $n = 1$, 2 oder 3 bedeuten, oder in einem höheren Alkohol oder Alkanoläther durchgeführt werden.

Geeignete Lösungsmittel der allgemeinen Formel IV und V sind z. B. Diglykoldimethyläther, Diglykoldiethyläther, Glykoldiethyläther, Ethylenglykol-mono-methyläther, -mono-ethyläther, -mono-propyläther, Diethylenglykol-mono-methyläther, -mono-propyläther. Bei Verwendung von Lösungsmitteln der allgemeinen Formel IV oder V oder in einem höheren Alkohol oder Alkanoläther kann die rohe Reaktionsmischung des Leuco-indophenols der Formel I direkt der Schwefelung unterworfen werden.

Wenn die Herstellung der Verbindungen der allgemeinen Formel I nach dem erfindungsgemäßen Verfahren in dem zweckmäßigen Temperaturbereich von 180 bis 280 °C oder in dem bevorzugten Temperaturbereich von 220 bis 260 °C in Gegenwart eines inerten Lösungsmittels durchgeführt wird, so sollte dieses bei Normaldruck zweckmäßigerweise einen Siedepunkt von 190 bis 290 °C bzw. 230 bis 270 °C oder höher, besitzen. Zweckmäßigerweise wird ein solches inertes Lösungsmittel ausgewählt, das mit Wasser bei Normaltemperatur nicht mischbar ist. Derartige inerte Lösungsmittel wirken bei der Umsetzung des Carbazolderivats II mit Verbindungen der Formel III, in denen $R^7$—OH ist, als azeotrope Schleppmittel für das bei der Reaktion abgespaltene Wasser. Derartige als azeotrope Schleppmittel geeignete Lösungsmittel sind beispielsweise Decalin, hochsiedende Benzine, Dichlorbenzol, Trichlorbenzol.

Bei der Verwendung eines inerten Lösungsmittels wird dessen Menge so gering wie möglich gehalten. In der Regel ist es nicht erforderlich, mehr als 15 Gew.% des inerten Lösungsmittels, bezogen auf das Gesamtgewicht der Reaktanten, einzusetzen.

Der Ausschluß von Sauerstoff bei der Herstellung der erfindungsgemäßen Verbindungen der Formel I wird z. B. dadurch bewirkt, daß die Reaktion unter der Atmosphäre eines Inertgases, wie z. B. Stickstoff, durchgeführt wird. Insbesondere wenn kein inertes Lösungsmittel verwendet wird, ist es zweckmäßig, mindestens die 1,5 fache stöchiometrische Menge, vorzugsweise die 2,5 fache stöchiometrische Menge der Verbindung der Formel III einzusetzen. Der eingesetzte Überschuß wirkt dabei als Lösungsmittel. Höhere Überschüsse als die 3 fache stöchiometrische Menge brauchen normalerweise nicht eingesetzt zu werden, stören jedoch nicht. Nach beendeter Reaktion kann in den meisten Fällen die überschüssige Verbindung der Formel III leicht, zweckmäßig unter vermindertem Druck, abdestilliert und in einem weiteren Ansatz wieder verwendet werden. Auch hier empfiehlt sich bei Einsatz von Verbindungen der Formel III, die eine OH-Gruppe aufweisen, die Extraktion mit wäßriger Alkalihydroxidlösung. Bei der Durchführung der Reaktion zwischen dem Carbazolderivat der allgemeinen Formel II und der Verbindung der Formel III kann der Reaktionsverlauf entweder dünnschichtchromatographisch und/oder bei $R^7 = —OH$ durch Bestimmung des abgespaltenen Wassers oder bei $R^7 = —NH_2$ durch titrimetrische Bestimmung des abgespaltenen Ammoniaks verfolgt werden.

Die erfindungsgemäße Herstellung der Indophenole bzw. Indamine der Formel I ist technisch einfach durchzuführen. Man arbeitet vor allem bei der bevorzugten Ausführung unter Zusatz von Jod mit ausgezeichneten Ausbeuten, insbesondere sehr guter Raum-Zeit-Ausbeute ; es bedarf keiner umständlichen Isolierungsmaßnahmen des Reaktionsproduktes, es entstehen praktisch keine Abwässer, Abfallösungsmittel oder sonstige ökologisch bedenklichen Abfallprodukte, und es können vor allem bei der bevorzugten Ausführung, unter Zusatz von Jod, Produkte von hoher Reinheit erhalten werden, die zur Herstellung von Schwefelfarbstoffen ausgezeichneter und stets reproduzierbarer Nuance und Farbreinheit dienen können. Im Hinblick auf die unterschiedliche Weiterverarbeitung der hergestellten Indophenole und Indamine der Formel I ist die Durchführung des erfindungsgemäßen Verfahrens ohne jedes Lösungsmittel am universellsten anwendbar.

Die Verbindungen der allgemeinen Formeln II und III sind bekannte, z. T. großtechnisch hergestellte

Produkte. Sofern sie nicht handelsüblich sind, lassen sie sich nach einfachen, bekannten Verfahren gut herstellen.

Beispiele für Aminocarbazole der Formel II sind : 3-Aminocarbazol, 3-Amino-9-ethylcarbazol, 3-Amino-9-butylcarbazol, 3-Amino-9-octylcarbazol. Beispiele für Hydroxy- bzw. Aminoverbindungen der Formel III sind : p-Phenylendiamin, N-Dimethyl-p-phenylendiamin, 2-Methyl-p-phenylendiamin, 1,4-Diaminonaphthalin, 1,5-Diaminonaphthalin, 1-Amino-4-hydroxynaphthalin, 1-Amino-5-hydroxynaphthalin, 1,4-Dihydroxynaphthalin, 1,5-Dihydroxynaphthalin, 4,4' Dihydroxydiphenyl, 4,4'-Dihydroxydiphenylsulfon, 4,4'-Dihydroxydiphenylamin, 4,4'-Dihydroxydiphenylsulfid.

Die folgenden Ausführungsbeispiele veranschaulichen die Herstellung und Verwendung der erfindungsgemäßen Schwefelfarbstoffe und die Herstellung der erfindungsgemäßen Zwischenprodukte der allgemeinen Formel I.

## Beispiel 1

a) Eine Mischung von 100 ml Diethylenglycol-mono-ethyläther, 90 g Schwefel, 71 g Natriumsulfid konz. (60 %ig) und 50 g des Carbazolindophenols der Formel

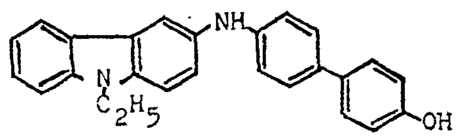

wird auf eine Temperatur von 137-139 °C erwärmt. Bei dieser Temperatur wird der Ansatz am Rückflußkühler 48 Stunden gerührt. Nach Ablauf dieser Zeit wird die heiße Schmelze in 2 l 60 °C warmes Wasser eingerührt. Die erhaltene Farbstoffanschlämmung wird filtriert, der Farbstoff auf dem Filter mit Wasser neutral gewaschen und getrocknet. Die Ausbeute beträgt 60 g Farbstoff.

Der erhaltene Farbstoff färbt Baumwolle aus der Dithionitküpe in einem vollen olivgrünen Farbton mit sehr guten Naßechtheiten und guter Lichtechtheit.

b) Das zur Herstellung des Farbstoffs eingesetzte Carbazolindophenol (3-(4 Hydroxydiphen-4'-ylamino)-9-ethylcarbazol) wird wie folgt hergestellt :

250 g 3-Amino-9-ethylcarbazol, 400 g 4,4'-Dihydroxydiphenyl und 6 g Jod werden 12 Stunden in der Schmelze unter Stickstoff bei 220-250 °C gerührt und das entstehende Reaktionswasser abdestilliert. Dann wird die Mischung abgekühlt, die erstarrte Schmelze fein pulverisiert und mit 5 %iger Natronlauge bei Zimmertemperatur 2 Stunden digeriert. Danach wird die Suspension abgesaugt, der Filterrückstand mit Wasser neutral gewaschen und getrocknet.

Die Ausbeute beträgt 325 g (~ 72,2 % d. Th.), der Schmelzpunkt ist 103-112 °C.

## Beispiel 2

a) Eine Mischung von 100 ml Diethylenglycol-mono-ethyläther, 90 g Schwefel, 71 g Natriumsulfid konz. (60 %ig) und 50 g des Leuco-Carbazolindamins der Formel

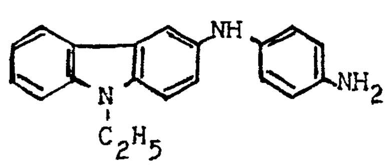

wird auf eine Temperatur von 137-139 °C erwärmt. Bei dieser Temperatur wird der Ansatz am Rückflußkühler 42 Stunden gerührt. Nach Ablauf dieser Zeit wird die heiße Schmelze in 2 l 60 °C warmes Wasser eingerührt.

Die erhaltene Farbstoffsuspension wird mit 100 g Natriumhydrogensulfit versetzt und 2 Stunden bei 70-80 °C gerührt. Anschließend wird der Farbstoff abgesaugt, mit Wasser neutral gewaschen und getrocknet. Ausbeute 72 g Farbstoff.

Der Farbstoff färbt auf Baumwolle aus der Dithionitküpe ein volles blaustichiges Grün mit sehr guten Naßechtheiten und guter Lichtechtheit.

b) Das in Absatz a) dieses Beispiels eingesetzte Leuco-Carbazolindamin (3-(4-Aminophenylamino)-9-ethylcarbazol) wird wie folgt hergestellt :

200 g 3-Amino-9-ethylcarbazol, 200 g p-Phenylendiamin und 4 g Jod werden unter Stickstoff als Schutzgas und unter Rühren so lange auf 220-250 °C erwärmt, bis die Ammoniakabspaltung praktisch beendet ist. Dann wird im Vakuum bei 0,2 bis 0,5 mbar der Überschuß des p-Phenylendiamins abdestilliert. Der Destillationsrückstand wird nach dem Erkalten fein pulverisiert. Die Ausbeute beträgt 231 g (~ 80 % d. Th.) ; der Schmelzpunkt liegt bei 78-85 °C.

Beispiel 3

a) Eine Mischung von 100 ml Diäthylenglycol-mono-ethyläther, 56 g Schwefel, 48 g Natriumsulfid konz. (60 %ig) und 50 g des Leuco-Carbazolindamins der Formel

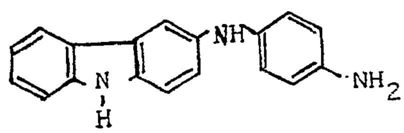

werden auf 138 °C erwärmt und bei dieser Temperatur 42 Stunden am Rückflußkühler gerührt. Dann wird die heiße Schmelze in 2 l 60 °C warmes Wasser eingerührt, die erhaltene Farbstoffsuspension mit 50 g Natriumhydrogensulfit versetzt, 2 Stunden bei 70-80 °C gerührt und abgesaugt. Der Farbstoff wird auf dem Filter mit Wasser neutral gewaschen und getrocknet. Die Ausbeute beträgt 52 g. Aus der Dithionitküpe färbt der Farbstoff Baumwolle in einem vollen blaugrünen Farbton. Die Färbung hat sehr gute Naßechtheiten und gute Lichtechtheit.

b) Das im Absatz a) dieses Beispiels eingesetzte Ausgangsmaterial (3-(4-Aminophenylamino)-carbazol) wird wie folgt hergestellt:

200 g 3-Aminocarbazol, 200 g p-Phenylendiamin und 4 g Jod werden unter Stickstoff als Schutzgas und unter Rühren so lange auf 220-250 °C erwärmt, bis die Ammoniakabspaltung praktisch beendet ist. Dann wird im Vakuum bei 0,2 bis 0,5 mbar der Überschuß des p-Phenylendiamins abdestilliert. Der Destillationsrückstand wird nach dem Erkalten fein pulverisiert. Die Ausbeute beträgt 300 g (100 % d. Th.); der Schmelzpunkt liegt bei 90-95 °C.

In analoger Weise, wie in den Abschnitten a) der vorangehenden Ausführungsbeispiele beschrieben, lassen sich die Carbazolindophenole und Carbazolindamine der folgenden Tabelle, die ihrerseits analog den Abschnitten b) der vorstehenden Ausführungsbeispiele hergestellt werden können, schwefeln.

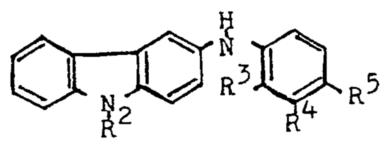

| Nr. | $R^2$ | $R^3$ | $R^4$ | $R^5$ | Nuance des geschwefelten Produkts auf Baumwolle |
|---|---|---|---|---|---|
| 4 | H | H | H | 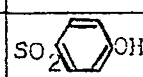 | oliv |
| 5 | H | H | $-CH_3$ | $-NH_2$ | grün |
| 6 | H | $\cdot CH=CH-CH=C<OH$ | | H | oliv |
| 7 | H | $\cdot CH=CH-CH=C<NH_2$ | | H | olivgrün |

**Ansprüche**

1. Neue Schwefelfarbstoffe, herstellbar durch Schwefeln neuer Leuco-Indophenole bzw. Leuco-Indamine der Formel I

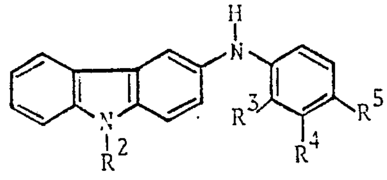

(I)

worin

$R^2$ Wasserstoff oder Alkyl mit 1 bis 8 C-Atomen,

$R^3$ und $R^4$ unabhängig voneinander Wasserstoff, oder Alkyl mit 1 bis 4 C-Atomen bedeuten oder gemeinsam ein Strukturelement der Formel Ia-d

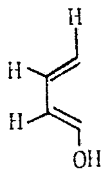 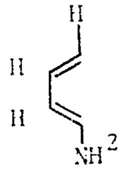 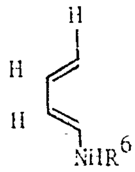 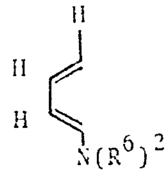

(Ia) (Ib) (Ic) (Id)

bilden,

$R^5$ Alkoxy mit 1 bis 4 C-Atomen, Amino, —$NHR^6$, —$N(R^6)_2$,

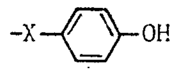

und, sofern $R^3$ und $R^4$ gemeinsam ein Strukturelement der Formel Ia-d bilden, auch Wasserstoff und, sofern $R^3$ und/oder $R^4$ ungleich Wasserstoff sind, auch Hydroxy, und

$R^6$ Alkyl mit 1 bis 4 C-Atomen bedeuten, wobei die für $R^2$, $R^3$, $R^4$ und $R^6$ stehenden Alkylreste gegebenenfalls durch ein Halogenatom substituiert sein können, und

X für —S—, —$SO_2$—, —NH— oder eine direkte Bindung steht, nach an sich bekannten Verfahren.

2. Neue Schwefelfarbstoffe gemäß Anspruch 1, wobei in dem Leuco-Indophenol bzw. Leuco-Indamin der Formel I

$R^2$ Wasserstoff oder Alkyl mit 1 bis 4 C-Atomen, eines der Symbole $R^3$ und $R^4$ Wasserstoff, das andere Wasserstoff, oder Alkyl mit 1 bis 4 C-Atomen bedeuten ; oder

$R^3$ und $R^4$ gemeinsam ein Strukturelement der Formel I a oder I b bilden, und $R^5$ Wasserstoff ist ; oder

$R^5$ Alkoxy mit 1 bis 4 C-Atomen, Amino, —$NHR^6$, —$N(R^6)_2$ oder

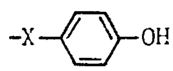

und

$R^6$ Alkyl mit 1 bis 4 C-Atomen bedeuten.

3. Neue Schwefelfarbstoffe gemäß den Ansprüchen 1 und 2, wobei die Verbindungen der Formel I nach dem an sich bekannten Verfahren der « Kochschmelze » geschwefelt werden.

4. Verfahren zur Herstellung neuer Schwefelfarbstoffe durch Umsetzung geeigneter Ausgangsmaterialien mit Schwefel und Alkalisulfid oder Alkalipolysulfid nach den an sich bekannten Verfahren der « Kochschmelze » oder der « Backschmelze », dadurch gekennzeichnet, daß als Ausgangsmaterialien Leuco-Indophenole bzw. Leuco-Indamine der Formel I des Anspruchs 1 eingesetzt werden.

5. Verfahren zur Herstellung neuer Schwefelfarbstoffe durch Umsetzung geeigneter Ausgangmaterialien mit Schwefel und Alkalisulfid oder Alkalipolysulfid nach dem an sich bekannten Verfahren der « Kochschmelze », dadurch gekennzeichnet, daß als geeignete Ausgangsmaterialien die neuen Leuco-Indophenole bzw. Leuco-Indamine der Formel I des Anspruchs 1 eingesetzt werden.

6. Neue Leuco-Indophenole bzw. Leuco-Indamine der Formel I

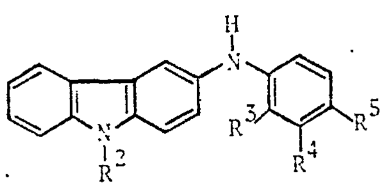

(I)

worin

$R^2$ Wasserstoff oder Alkyl mit 1 bis 8 C-Atomen,

$R^3$ und $R^4$ unabhängig voneinander Wasserstoff, oder Alkyl mit 1 bis 4 C-Atomen bedeuten oder gemeinsam ein Strukturelement der Formel Ia-d

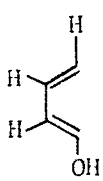 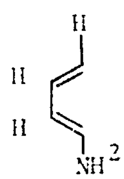 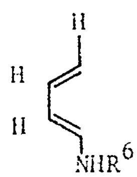 oder 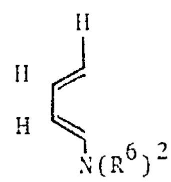

(Ia) (Ib) (Ic) (Id)

bilden,

$R^5$ Alkoxy mit 1 bis 4 C-Atomen, Amino, —$NHR^6$, —$N(R^6)_2$,

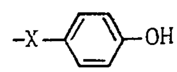

und, sofern $R^3$ und $R^4$ gemeinsam ein Strukturelement der Formel Ia-d bilden, auch Wasserstoff und, sofern $R^3$ und/oder $R^4$ ungleich Wasserstoff sind, auch Hydroxy,

$R^6$ Alkyl mit 1 bis 4 C-Atomen und

X —S—, —$SO_2$—, —NH— oder eine direkte Bindung bedeuten.

7. Neue Leuco-Indophenole bzw. Leuco-Indamine der Formel I, dadurch gekennzeichnet, daß $R^2$, $R^3$, $R^4$ und $R^5$ die in Anspruch 2 angegebenen Bedeutungen haben.

8. Verfahren zur Herstellung neuer Leuco-Indophenole bzw. Leuco-Indamine der Formel I

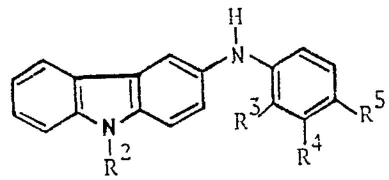

(I)

worin $R^2$, $R^3$, $R^4$ und $R^5$ die in Anspruch 4 angegebenen Bedeutungen haben, dadurch gekennzeichnet, daß ein 3-Aminocarbazol der allgemeinen Formel II

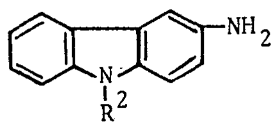

(II)

worin $R^2$ die in Anspruch 6 angegebenen Bedeutungen haben, mit einem Phenol bzw. Naphthol oder Aminobenzol bzw. Aminonaphthalin der Formel III

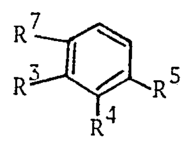

(III)

worin $R^7$ eine der Gruppen —OH oder —$NH_2$ ist und $R^3$, $R^4$ und $R^5$ die in Anspruch 4 genannten Bedeutungen haben, in mindestens stöchiometrischen Mengen unter Ausschluß von Sauerstoff auf Temperaturen von 150 bis 300 °C, vorzugsweise 220 bis 260 °C, erhitzt wird.

9. Verfahren nach Anspruch 8 dadurch gekennzeichnet, daß die Reaktion in Gegenwart katalytischer Mengen Jod durchgeführt wird.

10. Verwendung der Schwefelfarbstoffe der Ansprüche 1 bis 3 zum Färben von Zellulose und Polyamid.

**Claims**

1. New sulphur dyestuffs which can be prepared in accordance with processes which are in themselves known by sulphurising new leuco-indophenols or leuco-indamines of the formula I

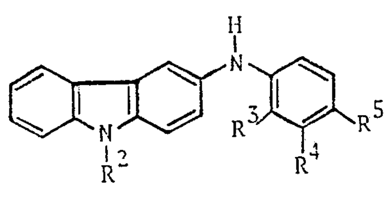

(I)

wherein

$R^2$ denotes hydrogen or alkyl having 1 to 8 C atoms,

$R^3$ and $R^4$ independently of one another denote hydrogen, halogen or alkyl having 1 to 4 C atoms, or conjointly form a structural element of the formula Ia-d

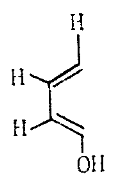 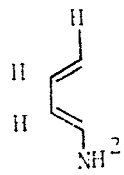 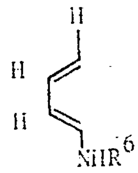 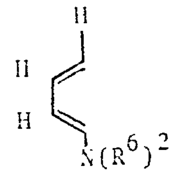

(Ia) (Ib) (Ic) (Id)

$R^5$ denotes alkoxy having 1 to 4 C atoms, amino, $—NHR^6$, $—N(R^6)_2$ and

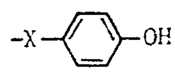

and also denotes hydrogen, if $R^3$ and $R^4$ conjointly form a structural element of the formula Ia-d, and also denotes hydroxyl if $R^3$ and/or $R^4$ are not hydrogen,

$R^6$ denotes alkyl having 1 to 4 C atoms, it being possible for the alkyl radicals which are represented by $R^2$, $R^3$, $R^4$ and $R^6$ to be substituted by a halogen atom, and

X denotes —S—, $—SO_2—$, —NH— or a direct bond.

2. New sulphur dyestuffs according to Claim 1, wherein, in the leuco-indophenol or leuco-indamine of the formula I,

$R^2$ denotes hydrogen or alkyl having 1 to 4 C atoms, and one of the symbols $R^3$ and $R^4$ denotes hydrogen and the other denotes hydrogen or alkyl having 1 to 4 C atoms ; or

$R^3$ and $R^4$ conjointly form a structural element of the formula Ia or Ib and $R^5$ is hydrogen ; or

$R^5$ denotes alkoxy having 1 to 4 C atoms, amino, $—NHR^6$, $—N(R^6)_2$ or

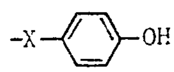

3. New sulphur dyestuffs according to Claims 1 and 2, wherein the compounds of the formula I are sulphurised by the « boil-melt » process, which is in itself known.

4. Process for the manufacture of new sulphur dyestuffs by reacting suitable starting materials with sulphur and an alkali metal sulphide or alkali metal polysulphide by the « boil-melt » or « bake-melt » processes, which are in themselves known, characterised in that the starting materials employed are leuco-indophenols or leuco-indamines of the formula I of Claim 1.

5. Process for the manufacture of new sulphur dyestuffs by reacting suitable starting materials with sulphur and an alkali metal sulphide or alkali metal polysulphide by the « boil-melt » process, which is in itself known, characterised in that the suitable starting materials employed are the new leuco-indophenols or leuco-indamines of the formula I of Claim 1.

6. New leuco-inophenols or leuco-indamines of the formula I.

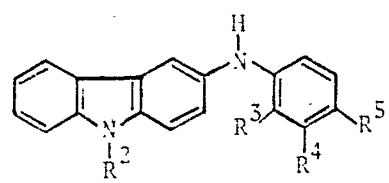 (I)

wherein

$R^2$ denotes hydrogen or alkyl having 1 to 8 C atoms,

$R^3$ and $R^4$ independently of one another denote hydrogen or alkyl having 1 to 4 C atoms, or conjointly form a structural element of the formula Ia-d

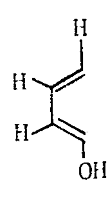 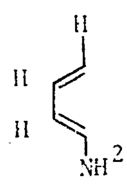 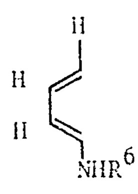 or 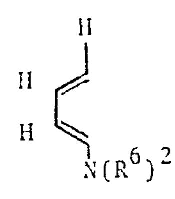

(Ia) (Ib) (Ic) (Id)

$R^5$ denotes alkoxy having 1 to 4 C atoms, amino, $—NHR^6$, $—N(R^6)_2$, and

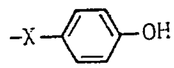

and also denotes hydrogen, if $R^3$ and $R^4$ conjointly form a structural element of the formula Ia-d, and also denote hydroxyl if $R^3$ and/or $R^4$ are not hydrogen,

$R^6$ denotes alkyl having 1 to 4 C atoms and

X denotes —S—, $—SO_2—$, —NH— or a direct bond.

7. New leuco-indophenols or leuco-indamines of formula I, characterised in that $R^2$, $R^3$, $R^4$ and $R^5$ have the meanings indicated in Claim 2.

8. Process for the manufacture of new leuco-indophenols or leuco-indamines of formula I

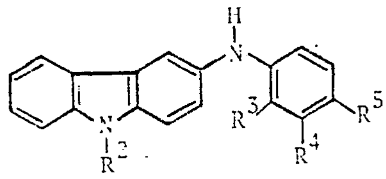

(I)

wherein $R^2$, $R^3$, $R^4$ and $R^5$ have the meanings given in Claim 4, characterised in that a 3-aminocarbazole of the general formula II

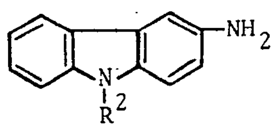

(II)

wherein $R^2$ has the meaning given in Claim 6, is heated with a phenol, naphthol, aminobenzene or aminonaphthalene of the formula III

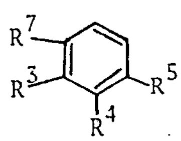

(III)

wherein $R^7$ is one of the groups —OH and $—NH_2$ and $R^3$, $R^4$ and $R^5$ have the meanings given in Claim 4, in at least stoichiometric quantities with exclusion of oxygen to temperatures of 150 to 300 °C, preferably 220 to 260 °C.

9. The process according to Claim 8, characterised in that the reaction is carried out in the presence of catalytic quantities of iodine.

10. Use of the sulphur dyestuffs of Claims 1 to 3 for dyeing cellulose and polyamide.

**Revendications**

1. Nouveaux colorants au soufre, préparables par sulfuration suivant des procédés connus de nouveaux leuco-indophénols ou de nouvelles leuco-indamines de formule I

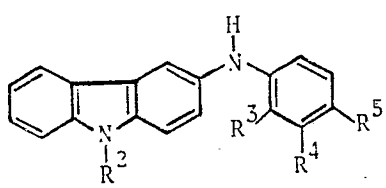

(I)

dans laquelle

$R^2$ représente l'hydrogène ou un alkyle en $C_1$-$C_8$,

$R^3$ et $R^4$ représentent chacun indépendamment l'un de l'autre, l'hydrogène ou un alkyle en $C_1$-$C_4$, ou bien forment ensemble un élément structurel de formule I, a à d

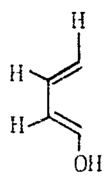 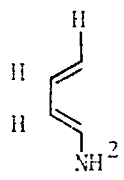 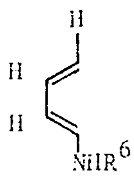 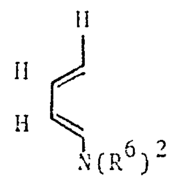

(Ia) (Ib) (Ic) (Id)

$R^5$ représente un alcoxy en $C_1$-$C_4$ ou un groupe amino, —$NHR^6$, —$N(R^6)_2$ ou

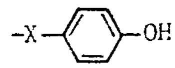

et, si $R^3$ et $R^4$ forment ensemble un élément de formule I, a à d, peut également être l'hydrogène, ou encore un hydroxyle si $R^3$ et/ou $R^4$ ne sont pas l'hydrogène,

$R^6$ étant un alkyle en $C_1$-$C_4$, les alkyles représentés par $R^2$, $R^3$, $R^4$ et $R^6$ pouvant être éventuellement substitués par un atome d'halogène, et

X désignant —S—, —$SO_2$—, —NH— ou une liaison directe.

2. Colorants au soufre selon la revendication 1, caractérisés en ce que dans le leuco-indophénol ou la leuco-indamine de formule I, $R^2$ est l'hydrogène ou un alkyle en $C_1$-$C_4$, parmi

$R^3$ $R^4$, l'un est l'hydrogène et l'autre l'hydrogène ou un alkyle en $C_1$-$C_4$, ou bien $R^3$ et $R^4$ forment ensemble un élément structurel de formule Ia ou Ib, et $R^5$ est l'hydrogène ; ou

$R^5$ est un alcoxy en $C_1$-$C_4$ ou un groupe amino, —$NHR^6$, —$N(R^6)_2$ ou

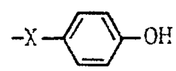

$R^6$ étant un alkyle en $C_1$ à $C_4$.

3. Nouveaux colorants au soufre selon la revendication 1 ou 2, les composés de formule I ayant été sulfurés par le procédé connu dit de « fusion-ébullition ».

4. Procédé de préparation de nouveaux colorants au soufre par réaction de corps de départ appropriés avec du soufre et un sulfure ou un polysulfure de métal alcalin, suivant les méthodes connues dites de « fusion-ébullition » ou « fusion-cuisson », procédé caractérisé en ce que les corps de départ employés sont les leuco-indophénols ou des leuco-indamines de formule I selon la revendication 1.

5. Procédé de préparation de nouveaux colorants au soufre par réaction de corps de départ appropriés avec du soufre et un sulfure ou un polysulfure de métal alcalin suivant la méthode connue dite de « fusion-ébullition », procédé caractérisé en ce que les corps de départ appropriés employés sont les nouveaux leuco-indophénols ou les nouvelles leuco-indamines de formule I selon la revendication 1.

6. Nouveaux leuco-indophénols et nouvelles leuco-indamines de formule I :

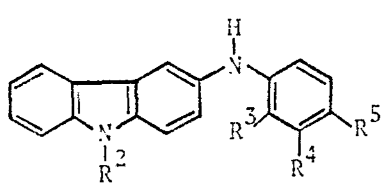

(I)

dans laquelle

$R^2$ représente l'hydrogène ou un alkyle en $C_1$-$C_8$,

$R^3$ et $R^4$ représentent chacun indépendamment l'un de l'autre, l'hydrogène ou un alkyle en $C_1$-$C_4$, ou bien forment ensemble un élément structurel de formule I, a à d

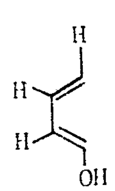 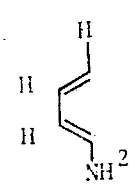 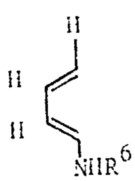 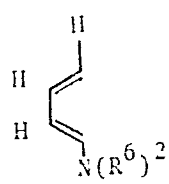

(Ia) (Ib) (Ic) (Id)

$R^5$ représente un alcoxy en $C_1$-$C_4$ ou un groupe amino, —$NHR^6$, —$N(R^6)_2$ ou

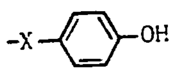

et, si $R^3$ et $R^4$ forment ensemble un élément de formule I, a à d, peut également être l'hydrogène, ou encore un hydroxyle si $R^3$ et/ou $R^4$ ne sont pas l'hydrogène,

$R^6$ étant un alkyle en $C_1$-$C_4$, et

X désignant —S—, —$SO_2$—, —NH— ou une liaison directe.

7. Nouveaux leuco-indophénols et nouvelles leuco-indamines de formule I selon la revendication 6, caractérisés en ce que les symboles $R^2$, $R^3$, $R^4$ et $R^5$ ont les significations données dans la revendication 2.

8. Procédé de préparation de nouveaux leuco-indophénols et de nouvelles leuco-indamines de formule I définies à la revendication 6, procédé caractérisé en ce que l'on chauffe entre 150 et 300 °C, de préférence entre 220 et 260 °C, à l'abri de l'oxygène, dans des proportions au moins stœchiométriques, un 3-aminocarbazole de formule générale II

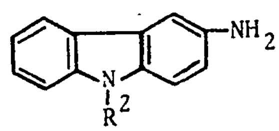

(II)

($R^2$ ayant la signification donnée à la revendication 6) avec un phénol ou un naphtol ou bien un aminobenzène ou un aminonaphtalène de formule III

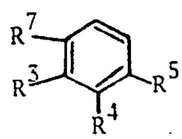

(III)

dans laquelle $R^7$ représente un groupe —OH ou —$NH_2$ et $R^3$, $R^4$ et $R^5$ ont les significations données à la revendication 6.

9. Procédé selon la revendication 8, caractérisé en ce que la réaction est effectuée en présence de quantités catalytiques d'iode.

10. L'utilisation des colorants au soufre selon l'une quelconque des revendications 1 à 3 pour la teinture de cellulose et de polyamides.